Europäisches Patentamt

**European Patent Office**

Office européen des brevets

(11) Veröffentlichungsnummer: **0 243 867 B1**

(12) **EUROPÄISCHE PATENTSCHRIFT**

(45) Veröffentlichungstag der Patentschrift: **13.03.91**

(51) Int. Cl.⁵: **A61K 31/725**

(21) Anmeldenummer: **87105907.7**

(22) Anmeldetag: **22.04.87**

(54) Mittel zur Verabreichung von Hyaluronsäure an Säuger.

(30) Priorität: **28.04.86 US 856732**

(43) Veröffentlichungstag der Anmeldung:
**04.11.87 Patentblatt 87/45**

(45) Bekanntmachung des Hinweises auf die
Patenterteilung:
**13.03.91 Patentblatt 91/11**

(84) Benannte Vertragsstaaten:
**AT BE CH DE ES FR GB IT LI NL SE**

(56) Entgegenhaltungen:
**EP-A- 0 143 393**
**WO-A-86/05984**

**CHEMICAL ABSTRACTS, Band 102, Nr. 11, 18 März 1985, Seiten 32,33, Zusammenfassung Nr. 89817s, Columbus, Ohio, US; MIYAZAKI, KYOSUKE et al.: "Studies on analgesic and anti-inflammatory effects of sodium hyaluronate (SPH)", & OYO YAKURI 1984, 28(6),1123-35**

**CHEMICAL ABSTRACTS, Band 106, Nr. 7, 16. Februar 1987, Seite 23, Zusammenfassung Nr. 43520u, Columbus, Ohio, US; E. TRABUC-CHI et al.: "Topical treatment of experimental skin lesions in rats: macroscopic, microsco-pic and scanning electron-microscopic eva-luation of the healing process", & INT. J. TISSUE REACT. 1986, 8(6),533-44**

(73) Patentinhaber: **MOBAY CORPORATION
Mobay Road
Pittsburgh Pennsylvania 15205-9741(US)**

(72) Erfinder: **Schultz, Richard H.
8088 Monrovia
Lenexa, KS 66215(US)**
Erfinder: **Wollen, Terry H.
Rt. 2, Box 60A
Wellsville, KS 66092(US)**
Erfinder: **Greene, Nathan D.
15463 Overbrook Lane
Leawood, KS 66224(US)**
Erfinder: **Brown, Karen K.
100000 N.E. Harrison Drive
Kansas City, MO 64155(US)**
Erfinder: **Mozier, John O., Dr.
10175 West 159th Street
Stanley, KS 66221(US)**

(74) Vertreter: **Schumacher, Günter, Dr.
c/o Bayer AG Konzernverwaltung RP Patent-abteilung
W-5090 Leverkusen 1 Bayerwerk(DE)**

**Beschreibung**

Die vorliegende Erfindung basiert auf dem Befund, daß ein bekanntes therapeutisches Arzeimittel, die Hyaluronsäure, ansich Mensch oder Pfed in wirksamer Weise mittels nachstehend definierter Techniken verabreicht werden kann, die die Vorgänge im Inneren des Körpers des Säugers erfordern, um dieses hochmolekulare Mittel zum Wirkungsort zu transportieren.

Konkret betrifft die Erfindung die Verwendung von Hyaluronsäure oder eines pharmakologisch unbedenklichen Salzes derselben zur Herstellung von intravenös, intrarnuskulär oder topisch verabreichbaren Arzneimitteln zur Behandlung von Arthritis in Gelenken von Pferden und Menschen.

Nach einer besonderen Ausführungsform ist die Erfindung dadurch gekennzeichnet, daß für die Herstellung einer Injektionslösung die Hyaluronsäure in Form einer wäßrigen Lösung von 0,5 bis 3,0 Gew.-% mit einer Viskosität von weniger als etwa 200 cST bei 37°C eingesetzt wird.

In einer weiteren Ausgestaltung ist die erfindungsgemäße Verwendung, dadurch ausgezeichnet, daß die Hyaluronsäure eine mittels FPLC bestimmte Molekulargewichtsverteilung aufweist, die nahezu vollständig zwischen 1,5 und $4x10^6$ u (1,5 und $4x10^6$ Dalton) liegt und ferner, daß die Hyaluronsäure nicht pyrogen ist und einen mittels UV-Extinktion bestimmten kombinierten Aminosäure- und Protein-Gehalt von weniger als etwa 1,25 mg/ml und einen Nucleinsäure-Gehalt von weniger als etwa 0,06 mg/ml aufweist.

Hyaluronsäure ist ein wohlbekanntes Mucopolysaccharid, das man im Gelenkgewebe und in der Glasflüssigkeit (Humor vitreus) des Auges von Säugern findet. Sie wird aus Hahnenkämmen und menschlichen Nabelschnüren sowie aus Bakterien-Kulturen wie denjenigen von Streptococcen der hämolytischen Gruppen A und C für verschiedene therapeutische Zwecke extrahiert. Die ursprüngliche therapeutische Verwendung dieses Stoffes war diejenige als Ersatz für die Glasflüssigkeit des menschlichen Auges zur Unterstützung der Augenchirurgie, insbesondere bei der Behandlung der Netzhaut-Ablösung. Er findet ebenfalls Anwendung zur Linderung von Trauma oder Reizung in Gelenk-Gewebe von Säugern einschließlich des Menschen durch Injektion in die synoviale Gelenkflüssigkeit. Es wurde vorgeschlagen, sie als primäres Medikament sowie auch als Hilfsstoff zusammen mit anderen Gelenk-Medikamenten einzusetzen. Eine ausführliche Diskussion ihrer verschiedenen Anwendungsmöglichkeiten findet sich in der US-PS 4 141 973.

Der Einsatz von Hyaluronsäure für sich allein und mit Cortison in verschiedenen Gelenken von Tieren, insbesondere von Pferden, wird in dem Artikel "Effect of Intra-articular Injection of Hyaluronic Acid on the Clinical Symptoms of Osteoarthritis and on Granulation Tissue Formation" von Rydell et al., erschienen auf den Seiten 25 bis 32 der Ausgabe (Nr. 80) vom Oktober 1971 von Clinical Orthopaedics and Related Research, diskutiert. Über die Verwendung von Hyaluronsäure in menschlichen Gelenken wird berichtet in dem Artikel "Preliminary Clinical Assessment of Na Hyaluronate Injection into Human Arthritic Joints" von Peyron et al., erschienen auf den Seiten 731 bis 736 von Band 22 , Nr. 8, Oktober 1974, von Pathologie Biologie. Schließlich wird über den Einsatz von Hyaluronsäure bei der Verringerung fibrotischer Wundreaktion in "Decreased Granulation Tissue Reaction After Installment of Hyaluronic Acid" von Rydell, erschienen auf den Seiten 307 bis 311 von Band 41 der Acta Orthop. Scandinav., berichtet.

Die intra-artikuläre Anwendung von Hyaluronsäure in Pferde-Gelenken wurde wirtschaftlich vorangetrieben in Verbindung mit den Produkten Hylartil® und Hylartin V® von Pharmacia und Synacid von Sterivet. Die wirtschaftliche Attraktivität hielt sich jedoch aufgrund der Notwendigkeit, diese Produkte mittels Injektion in das erkrankte Gelenk zu verabreichen, in Grenzen.

Ein verwandtes Material, ein polysulfatiertes Glycosaminoglycan, wurde kürzlich auf dem US-Markt von Luitpold Pharmaceuticals unter der Handelsbezeichnung Adequan® (auch in Europa bekannt als Arteparon®) für die Behandlung arthritischer Gelenke bei Pferden eingeführt. Anfangs war der empfohlene Verabreichungsweg intra-artikulär mit einer Injektion von 250 mg pro Woche während einer Dauer von 5 Wochen. Auf den Seiten 446 und 447 der Ausgabe vom April 1984 von Veterinary Medicine wird vorgeschlagen, daß dieses Material intramuskulär in eta der doppelten Dosis in Intervallen von vier Tagen während einer Dauer von etwa 4 bis 5 Wochen verabreicht werden kann.

Es ist berichtet worden, daß polysulfatierte Glycosaminoglycane die Biosynthese von Hyaluronsäure in den synovialen Membranen der Kniegelenke von Kaninchen stimulieren, was den Gedanken nahelegt, daß, obwohl der Wirkungsmodus von dem der Hyaluronsäure verschieden sein mag, die gleichen Zustände in günstiger Weise hervorgerufen werden könnten. Interessanterweise gibt der Bericht "Influences of Sulfated Glycosaminoglycans on Biosynthesis of Hyalu ronic Acid in Rabbit Knee Synovial Membrane" von Nishikawa et al., erschienen auf den Seiten 146 bis 153 der Ausgabe vom Juli 1985, Band 240 , von Arch. Biochem. Biophys., an, daß Hyaluronsäure selbst keinen derartigen stimulierenden Effekt aufweist.

Es wird berichtet, daß das stimulierende Mittel wirksam ist, wenn es entweder intramuskulär an Menschen oder subkutan an Ratten verabreicht wird. Der erstere Effekt wird aufgezeigt in "Vergleich von Glykosaminoglykanpolysulfat (Arteparon) und physiologischer Kochsalzlösung bei Arthrosen großer Gelenke. Ergebnisse einer multizentrischen Doppelblindstudie" von Siegmeth et al., erschienen auf den Seiten 223 bis 228 der Ausgabe vom Juli/August 1983 (Band 42 , Nr. 4) der Z. Rheumatol.. Der letztere Effekt wird diskutiert in "Die tierexperimentelle Gonarthrose der Ratte und ihre Therapie mit Glyko-amino-glykan-polysulfat" von Buchmann et al., erschienen auf den Seiten 100 bis 107 der Ausgabe 1985 (Band 44 , Nr. 3) der Z. Rheumatol..

Es wird auch berichtet, daß das stimulierende Mittel eubeb breiten Bereich niederer Molekulargewichte aufweist. In dem Artikel "Influence of a Glycosaminoglycan Polysulfate (Arteparon) on Lysosomal Enzyme Release from Human Polymorphonuclear Leucocytes" von Mikulikova, erschienen auf den Seiten 50 bis 53 der Ausgabe März/April 1982 (Band 41 , Nr. 2) der Z. Rheumatol., findet sich eine Angabe, daß Arteparon in Anteile fraktioniert werden kann, die Molekulargewichte zwischen 3 000 und 17 000 haben. In "Polysulfated Glycosaminoglycan: a New Intra-articular Treatment for Equine Lameness" von Hamm, Goldmann und Jones, erschienen auf den Seiten 811 bis 816 der Ausgabe vom Juni 1984 von Veterinary Medicine wird mitgeteilt, daß Adequan ein angenähartes mittleres Molekulargewicht von 10 000 besitzt.

Das polysulfatierte Glycosaminoglycan wird mit einer Dosis-Empfehlung von 250 mg zur intra-artikulären Verabreichung an Pferde eingesetzt. Hyaluronsäure wird mit einer empfohlenen Dosis von 20 mg zur intraartikulären Verabreichung eingesetzt. Obwohl berichtet worden ist, daß polysulfatiertes Glycosaminoglycan intramuskulär mit der doppelten Dosierung (500 mg) verabreicht werden kann, war ein ähnlicher Effekt für hochmolekulares Natrium-hyaluronat nicht zu erwarten.

Es war nicht erwarten, daß Natrium-hyaluronat einer Fernverabreichung (Verabreichung entfernt vom vorgesehenen Wirkungsort) zugänglich sei, da angenommen wurde, daß seine primäre Wirkung in der Schmierwirkung besteht, und wegen seines Molekulargewichts, das typischerweise gut oberhalb von 1 x $10^6$ u (1 x $10^6$ Dalton) liegt. Es war vielmehr zu erwarten, daß Moleküle mit einem solchen Molekulargewicht zu groß sein würden für eine Überführung durch Säuger-Gewebe hindurch zum entfernten Ort des Traumas. Somit ging man davon aus, daß bei einer Fernverabreichung vernünftig dimensionierter Dosen wirksame Mengen Natrium-hyaulronat nicht durch den Körper des Säugers zum Wirkungsort transportiert werden können.

Nunmehr wurde gefunden, daß die Fernverabreichung von Hyaluronsäure zur Verminderung des Schmerzes und der Schwellung von traumatisiertem oder gereiztem Säuger-Gewebe, insbesondere von Gelenk-Gewebe, wirksam ist.

Ein Verfahren zur Verminderung der Folgeerscheinungen des Traumas in gereiztem oder entzündetem Säuger-Gewebe durch Fernverabreichung von Hyaluronsäure oder einem pharmakologisch unbedenklichen Salz derselben wurde entwickelt. (Im Folgenden wird aus Gründen der Vereinfachung der Begriff "Hyaluronsäure" austauschbar zur Bezeichnung sowohl der freien Säure als auch ihrer pharmakologisch unbedenklichen Salze benutzt, ausgenommen dort, wo etwas Anderes ausdrücklich vermerkt ist). Die Hyaluronsäure wird in den Körper des Säugers an einem anderen Ort als dem Ort des traumatisierten Gewebes eingeführt und wird durch die Prozesse im Inneren des Körpers in wirksamer Weise zu dem Wirkungsort transportiert. Dies ermöglicht die Ausnutzung sol cher bequemer Verabreichungswege wie der intramuskulären, intravenösen, subkutanen Injektion und der topischen Applikation.Zwei besonders bevorzugte Wege der Verabreichung sind die intramuskuläre Injektion und die topische Applikation in einem anerkannten transdermalen Träger, und ein einer solchen Behandlung besonders zugänglicher Zustand ist gereiztes oder entzündetes Gelenk-Gewebe.

Fig. 1 zeigt eine Reihe graphischer Darstellungen der Veränderung des Umfangs des Karpalgelenks mit der Zeit nach der Behandlung mit Hyaluronsäure für behandelte Pferde und Kontroll-Pferde bei durch Freund's Complete Adjuvant induziertem Trauma im Karpalgelenk.

Fig. 2 zeigt eine Reihe graphischer Darstellungen der Veränderung des Bewegungsbereichs (ROM) mit der Zeit nach der Behandlung für dieselben Pferde wie in Fig. 1.

Fig. 3 zeigt eine Reihe graphischer Darstellungen der Veränderung des Bewegungsbereichs (ROM), normalisiert auf den Tag der Behandlung, mit der Zeit nach der Behandlung für dieselben Pferde wie in Fig. 1.

Fig. 4 zeigt die gleiche Reihe graphischer Darstellungen wie die Fig. 3, wobei jedoch sämtliche Ordinatenwerte willkürlich um 23,7 % erhöht wurden, um den niedrigsten Punkt auf 0 % zu setzen.

Fig. 5 zeigt eine Reihe graphischer Darstellungen des Lahmheits-Index mit der Zeit nach der Behandlung für dieselben Pferde wie in Fig. 1.

Fig. 6 zeigt eine Reihe graphischer Darstellungen der prozentualen Verbesserung des Schreitens mit der Zeit nach der Behandlung für dieselben Pferde wie in Fig. 1.

Das Trauma in gereiztem oder entzündetem Säuger-Gewebe wird dadurch verringert, daß Hyaluronsäure mittels irgendeines der eingeführten Wege der Verabreichung, ausgenommen die direkte Anwendung auf das erkrankte Gewebe, verabreicht wird. Eine besonders interessante Ausführungsform betrifft die Behandlung von Gelenk-Gewebe. Die direkte Verabreichung der Hyaluronsäure schließt die intra-artikuläre Injektion ein, die ein Vorgang ist, der bei den größeren Gelenken der größeren Säuger, etwa den Beingelenken von Pferden, beträchtliche Sorgfalt und Geschicklichkeit erfordert. Die Behandlung kleinerer Säugetiere wie Hunde und Katzen und kleinerer Gelenke größerer Säuger wie etwa der Fingergelenke des Menschen erfordert eine noch entsprechend größere Sorgfalt und Geschicklichkeit. Die Behandlung mittels Fernverabreichung wie etwa durch intramuskuläre, intravenöse oder subkutane Injektion oder topische Anwendung in einem transdermalen Träger ist in solchen Fällen sehr viel bequemer und attraktiver. Die Tatsache, daß die inneren Transportsysteme des Säugerkörpers so arbeiten, daß sie Hyaluronsäure an die erkrankten Stellen transportieren, macht es möglich, ebenso auch anderes traumatisiertes Gewebe durch Fernverabreichung zu behandeln. So kann die Fernverabreichung eingesetzt werden zur Behandlung anderer Zustände, bei denen Hyaluronsäure Andwendung gefunden hat, etwa bei Adhäsionen nach chirurgischen Eingriffen in Verbindung mit Einschnitten und Sehnen-Reparaturen, über die in dem Artikel von Rydell et al., erschienen auf den Seiten 25 bis 32 der Ausgabe vom Oktober 1972 von Clinical Orthopedics and Related Research, berichtet ist.

Die bei der Behandlung von gereiztem oder entzündetem Gewebe durch Fernverabreichung einsetzbare Hyaluronsäure kann eine solche eines beliebigen Typs sein, der als geeignet für solche Zwecke anerkannt ist. Sie kann aus Tiergewebe wie Hahnenkämmen oder Nabelschnüren oder aus Bakterien-Kulturen wie denjenigen von Streptococcen der hämolytischen Gruppen A und C extrahiert werden. Sie sollte rein genug sein, um das Hervorrufen einer nachteiligen oder toxischen Reaktion in dem in Behandlung befindlichen Säuger-Gewebe zu vermeiden. Das bedeutet, daß sie frei von Pyrogenen ist und einen hinreichend niedrigen Gehalt an Proteinen und Nucleinsäuren aufweist, daß keine nennenswerte Immunreaktion ausgelöst wird. Sie ist vorzugsweise von hohem Molekulargewicht und hat vo rzugsweise auch eine niedrige Viskösität zur Verabreichung über die Wege der Injektion. Das Polymer kann in Form seiner freien Säure oder in Form eines beliebigen, pharmakologisch unbedenklichen Salzes vorliegen.

Die bevorzugte Quelle für Hyaluronsuaure ist eine Kultur eines geeigneten Mikroorganismus. Die Anwendung der der in der EP-A- 0 144 019 beschrieben Arbeitsmethoden des Kultivierens und Erntens sind besonders wertvoll für die Gewinnung eines Materials mit der gewünschten Reinheit und dem gewünschten Molekulargewicht. Von den Organismen, bei denen sich diese Techniken anwenden lassen, werden die Streptococcen der Gruppen A und C bevorzugt, wobei diejenigen der Gruppe C besonders bevorzugt werden und Streptococcus equi am meisten bevorzugt wird. Weiter bevorzugt wird die Hyaluronsäure, die gemäß den Lehren der gleichzeitig anhängigen US-Patentanmeldung Serial No. 816 548, eingereicht am 6. Januar 1986, erhalten wird.

Sowohl der Gehalt an Proteinen und Aminosäuren als auch der Gehalt an Nucleinsäuren der Hyaluronsäure sollte sorgfältig kontrolliert und gesteuert werden, da bekannt ist, daß beide antigene Aktivität in Säugern entfalten.

Der Gehalt an beiden wird zweckmäßigerweise mit Hilfe der US-Extinktion überwacht und bewertet, wobei der Gehalt an Proteinen mit der optischen Dichte bei 280 nm und der Gehalt an Aminosäuren mit der optischen Dichte bei 257 nm korreliert. Vorzugsweise betragen ersterer weniger als etwa 1,25 mg/ml, insbesondere weniger als etwa 0,1 mg/ml, und letzterer weniger als etwa 0,6 mg/ml, insbesondere weniger als etwa 0,005 mg/ml. In dieser Hinsicht unterscheidet die Extinktion bei 280 nm nicht zwischen Aminosäuren und Proteinen. Während jedoch Aminosäuren allein nicht antigen sind, komplexieren sie ohne weiteres mit Hyaluronsäure, und der Komplex vermag leicht eine Immunantwort in Säugern auszulösen. Aus diesem Grunde ist es im Zusammenhang mit der vorliegenden Technik wünschenswert, den Gehalt an beiden zu steuern, und es ist sinnvoll, einen Maximal-Gehalt für die Kombination aus beiden spezielle anzugeben, der mit einer besonderen UV-Extinktion korreliert. In einer besonders bevorzugten Hyaluronsäure beträgt der Gesamt-Aminosäure-Gehalt, gemessen mittels der o-Phthalaldehyd-Fluoreszenz-Technik (die zwangsläufig mit der Hydrolyse des etwa vorhandenen Proteins bis zurück zu den Aminosäuren, aus denen es besteht, verbunden ist), weniger als etwa 0,4 mg/ml, und der Nucleinsäure-Gehalt ist kleiner als etwa 0,06 mg/ml, gemessen mittels der Ethidiumbromid-Fluoreszenz-Technik.

Die Hyaluronsäure kann in ihrer Form als freie Säure oder in Form irgendeines beliebigen, pharmakologisch unbedenklichen Salzes zur Anwendung gelangen. Eine der zweckmäßigsten Formen ist diejenige des Natrium-Salzes, da dieses Polymer typischerweise durch nacheinander erfolgende Fällungen in Ethanol oder anderen organischen Lösungsmitteln und Auflösen in Wasser gereinigt wird, und das Natrium-Salz sich für solche Arbeitsweisen besonders eignet. In der Tat wurden sämtliche hierin diskutierten Grenzwete in bezug auf die Reinheit, die Viskosität und das Molekulargewicht am Natrium-Salz entwickelt, und das gilt auch für die im Folgenden erörterten spezifischen Anwendungsdaten. Die Fernverabreichung ist jedoch in gleicher Weise auch auf andere Formen wie die freie Säure und das Kalium-Salz anwendbar. Aus Gründen

EP 0 243 867 B1

der Vereinfachung faßt die Diskussion alle diese Formen unter dem Begriff Hyaluronsäure zusammen.

Die Hyaluronsäure sollte ein hohes mittleres Molekulargewicht haben. Wenngleich Formen dieses Stoffes mit mittleren Molekulargewichten von 55 000 oder weniger bekannt sind, hat die bevorzugte Hyaluronsäure ein mittleres Molekulargewicht von wenigstens $5 \times 10^5$, bestimmt mittels FPLC (Fast Protein Liquid Chromatography) gemäß der Technik, die in der gleichzeitig anhängigen US-Patentanmeldung Serial No. 816 548, eingereicht am 6. Januar 1986, offenbart ist. Mittlere Molekulargewichte oberhalb von etwa 1,0 $\times 10^6$, vorzugsweise 1,2 x $10^6$ und insbesondere oberhalb von etwa 1,8 x $10^6$, sind besonders bevorzugt. Es wird weiter bevorzugt, daß die Hyaluronsäure eine verhältnismäßig enge Molekulargewichts-Verteilung zeigt, und eine Verteilung mit nur einem einzigen Peak der Gel-permeation wird besonders bevorzugt. Eine Molekulargewichtsverteilung mit einem symmetrischen FPLC-Peak, bei dem 98 % der Moleküle ein Gewicht zwischen etwa 1,2 x $10^6$ und 4,0 x $10^6$ haben, wird besonders bevorzugt.

Die Hyaluronsäure kann entweder eine hohe oder eine niedrige Viskosität besitzen, je nach Zweckmäßigkeit für den gewünschten Weg der Verabreichung. Die höheren Viskositäten sind für topische Anwendungen zweckmäßig, während die niedrigeren Viskositäten sich besonders für die Verabreichungswege per Injektion eignen, d.h. die intramuskuläre, intravenöse oder subkutane Verabreichung. Die höhermolekulare Hyaluronsäure kann vorteilhafterweise Viskositäten zwischen etwa 900 und 5000 cSt bei 37 °C für topische Anwendungen und vorteilhafterweise Viskositäten von weniger als etwa 500 cSt, vorzugsweise weniger als etwa 150 cSt, bei 37 °C für andere Wege der Verabreichung aufweisen. In beiden Fällen wird die Viskosität zweckmäßig gemessen an einer wäßrigen Lösung mit 1 Gew.-% des Natrium-Salzes in einem Cannon-Manning-Semi-Micro-Viscosimeter entsprechend den Vorschriften in ASTM D 445 und D 2515. Das niedrigviskose Material erleichtert in hohem Maße die Verabreichung auf den Wegen der Injektion, da es beispielsweise den Einsatz einer angemessen konzentrierten wäßrigen Natriumhyaluronat-Lösung in der Praxis angepaßten Dosierungen ermöglicht. So läßt sich eine 1-proz. wäßrige Lösung von Natriumhyaluronat in einfacher Weise für Injektions-Dosen von etwa 10 ml einsetzen, die etwa 100 mg des Wirkstoffs enthalten, sofern seine Viskosität kleiner als etwa 200 cSt bei 37 °C ist.

Die Behandlung gereizten oder entzündeten Säuger-Gewebes mittels Fernverabreichung erfordert eine Behandlungsvorschrift in bezug auf die Dosis oder Total-Dosis, die dahingehend wirksam ist, daß sie das Trauma vermindert oder lindert. Vorzugsweise werden wenigstens etwa 0.02 mg Hyaluronsäure pro 1 lb. des Körpergewichts des der Behandlung unterzogenen Säugers verabreicht, was etwa 0.044 mg/kg entspricht. Es wird besonders bevorzugt, wengistens etwa 0,088 mg/kg (0,04 mg/lb.) und insbesondere 0,176 mg/kg (0,08 mg/lb.), bezogen auf das Körpergewicht, zu verwenden. Im Falle der topischen Anwendung ist es besonders erwünscht, mehr als etwa 0,22 mg/kg, (0,10 mg/lb.), insbesondere etwa 0,33 mg/kg (0,15 mg/lb.), bezogen auf das Körpergewicht, anzuwenden.

Da Hyaluronsäure eine in Säugern natürlich vorkommende Substanz ist, wird angenommen, daß es keinen naturgegebenen oberen Grenzwert für die erträgliche Dosis gibt. Wie bei allen medizinischen Behandlungen entspricht es jedoch einem Gebot der Klugheit und Vorsicht, nicht mehr zu verwenden, als zur Erzielung der angestrebten Wirkung erforderlich ist. Weiterhin besteht die Gefahr, daß Verunreinigungen, die in hinreichend niedrigen Konzentrationen vorliegen, so daß sie bei den wirksamen Dosierungen gut vertragen werden, bei ungerechtfertigt hohen Dosierungen schädliche Reaktionen hervorrufen können.

Die topische Behandlung sollte in Form einer kombinierten Anwendung der Hyaluronsäure mit einem verträglichen transdermalen Träger durchgeführt werden. Jeder anerkannte Träger wie Methylsalicylat, Natriumsalicylat, Benzylalkohol, Ölsäure, 10-proz. Propylengylcol, 1-proz. Natriumgylcolat, 1-proz. Polyoxyethylen-10-cetylether, 0,1-proz. Natrium-EDTA, 1-proz. Natriumdodecylsulfat oder Dimethylsulfoxid (DMSO) ist geeignet, wobei DMSO besonders bevorzugt wird. Eine geeignete Formulierung zur Applikation ist eine Mischung aus einer wäßrigen Lösung von weniger als etwa 3 Gew.-% Hyaluronsäure, insbesondere als Natriumhyaluronat, mit eine wirksamen Menge eines transdermalen Trägers. Eine bevorzugte Formulierung betrifft eine wäßrige Lösung mit zwischen etwa 0,5 und 2,5 Gew.-% Hyaluronat und bis zu etwa 30 Vol.-% des transdermalen Trägers. Die Hyaluronat-Lösung und die Gesamt-Formulierung zeigen beide vorteilhafterweise Viskositäten oberhalb von etwa 1000 cSt bei 37 °C.

Es wurde gefunden, daß die vorliegende Behandlung besonders wirksam ist bei der Behandlung von Gliederschmerzen großer Säuger, einschließlich solcher Schmerzen, die durch arthritische Zustände erzeugt werden. Besonders bevorzugte Anwendungsformen betreffen die Behandlung von Pferden und Menschen durch intramuskuläre Injektion und topische Verabreichung in einem transdermalen Träger. Eine besonders wirksame Behandlung von Gliederschmerzen in den Beingelenken, insbesondere in dem Karpal-Gelenk und in dem Tibiotarsal-Gelenk (oder Sprunggelenk), von Pferden ist eine intramuskuläre Injektion, vorzugsweise in den Halsmuskel. Eine besonders wirksame Behandlung von Muskel-Skelett-Schmerzen beim Menschen ist die topische Anwendung in einem transdermalen Träger wie Dimethylsulfoxid (DMSO).

5

Die Applikation kann in der Nachbarschaft des erkrankten Gelenk-Gewebes, jedoch auch in beträchtlicher Entfernung von diesem, erfolgen.

Die Arbeitstechniken der Fernverabreichung der vorliegenden Erfindung lassen sich auch zur Linderung anderer Zustände anwenden, gegen die Hyaluronsäure anerkanntermaßen wirksam ist. Hierzu zählen die Verringerung oder Verhütung von Adhäsionen an Stellen chirurgischer Eingriffe, insbesondere chirurgischer Eingriffe, von denen Sehnen betroffen sind.

Die Arbeitstechniken der Fernverabreichung der vorliegenden Erfindung sind von besonderem Interesse im Hinblick auf solche Säugetiere, die allgemeine als Gefährten des Menschen betrachtet werden. Die Besserung von Schmerzen oder Unwohlsein dieser tierischen Gesellschafter des Menschen ist von größtem Interesse und von höchster praktischer Bedeutung unter allen der Behandlung prinzipiell zugänglichen Säugetieren. Von besonderem Interesse sind in dieser Gruppe Katzen, Hunde und Pferde.

Die vorliegende Erfindung wird durch die folgenden Beispiele näher erläutert, ist jedoch nicht auf diese beschränkt.

Beispiel 1

Untersuchung der intramuskulären Behandlung von Pferden

Eine Untersuchung an acht Stuten und Wallachen aus gemischter Zucht wurde durchgeführt, um zu bestimmen, ob die intramuskuläre Verabreichung von Natriumhyaluronat in der Lage ist, die durch die intra-artikuläre Injektion von Freund's Complete Adjuvant in das Interkarpalgelenk induzierten Symptome zu lindern. Hierbei handelt es sich um ein allgemeines und wohlakzeptiertes Modell zur Untersuchung von Gliederschmerzen, insbesondere arthritischen Zuständen, bei Pferden. Die Untersuchung ergab, daß drei intramuskuläre Injektionen von 0,176 mg/kg (0,08 mg/lb.), bezogen auf das Gewicht des behandelten Pferdes, in den Halsmuskel 5, 9 und 13 Tage nach der Auslösung des Gliederschmerzes mit einer 0,7 ml-Injektion des Adjuvans wirksam waren.

Die acht Pferde wurden zunächst mehrer Tage an die Anlagen der Stallungen und der Prüfgeräte gewöhnt und danach zur Aufstellung einer Basis-Bewertung in bezug auf die Test-Kriterien Gelenkumfang, Bewegungsbereich, Schreiten und Lahmheit untersucht. Am folgenden Tag erhielten alle acht Pferde eine Injektion von 0,7 ml Freund's Complete Adjuvant in das linke Intercarpal-Gelenk. Die Pferde zeigten während der nächsten vier Tage Schmerzen im linken Vorderbein. Am vierten Tag nach der intra-artikulären Injektion des Reizmittels wurden die Pferde erneut in bezug auf die gleichen vier Kriterien untersucht. Für jedes Pferd wurde eine Gesamt-Bewertung entwickelt, die aus der Summe der Differenzen zwischen den beiden Meßwerten jedes Parameters (ausgenommen das Schreiten) bestand. Diese Werte wurden dann dazu benutzt, vier Pferde einer Kontrollgruppe und vier Pferde einer Behandlungsgruppe in der Weise zuzuordnen, daß das Pferd mit dem höchsten Wert der einen Gruppe, die Pferde mit den beiden nächsthöheren Werten der anderen Gruppe, die Pferde mit d en beiden folgenden nächsthöheren Werten der ersten Gruppe, die zwei Pferde mit den darauf folgenden Werten der zweiten Gruppe und das letzte Pferd der ersten Gruppe zugeordnet wurden. Die erste Gruppe wurde durch willkürliches Werfen einer Münze als Kontrollgruppe bestimmt. Die Meßtabelle sah aus, wie folgt, wobei die Meßtage willkürlich als -6 und -1 bezeichnet wurden.

6

## Tabelle

| Pferd Nr. | Gelenkumfang | | | Bewegungsbereich | | |
|---|---|---|---|---|---|---|
| | -6 | -1 | Diff. | -6 | -1 | Diff. |
| 1 | 31,9 | 36,2 | 4,3 | 155° | 65° | 90° |
| 2 | 29,4 | 34,4 | 5,0 | 150° | 55° | 95° |
| 3 | 29,3 | 33,5 | 3,7 | 160° | 30° | 130° |
| 4 | 30,0 | 35,5 | 5,5 | 195° | 45° | 100° |
| 5 | 29,3 | 33,4 | 3,6 | 150° | 45° | 105° |
| 6 | 31,5 | 36,2 | 4,7 | 190° | 35° | 105° |
| 7 | 30,5 | 36,0 | 5,5 | 150° | 55° | 95° |
| 8 | 31,0 | 34,5 | 3,5 | 150° | 60° | 90° |

- wird fortgesetzt -

## Tabelle - Fortsetzung

| Pferd Nr. | Lahmheit | | | | Schreiten | | | |
|---|---|---|---|---|---|---|---|---|
| | -6 | -1 | Diff. | Total | -6 | -1 | Diff. | Total |
| 1 | 0 | 4 | 4 | 98,3 | 157,1 | 138,5 | 18,2 | 116,5 |
| 2 | 0 | 5 | 5 | 105,0 | 161,5 | 76,1 | 85,4 | 190,4 |
| 3 | 0 | 5 | 5 | 138,7 | 151,1 | 78,5 | 82,8 | 221,5 |
| 4 | 0 | 5 | 5 | 110,5 | 160,4 | 0 | 160,4 | 270,9 |
| 5 | 0 | 4 | 4 | 112,6 | 163,1 | 126,7 | 36,4 | 149,0 |
| 6 | 0 | 4 | 4 | 113,7 | 161,3 | 145,7 | 15,6 | 134,2 |
| 7 | 0 | 5 | 5 | 105,5 | 145,4 | 100,9 | 44,5 | 150,0 |
| 8 | 0 | 5 | 5 | 98,5 | 157,9 | 114,7 | 40,2 | 138,7 |

| Rang | Pferd Nr. | | Gruppe Kontrolle I | | Gruppe Behandlung II | |
|---|---|---|---|---|---|---|
| 1 | 3 | 138,7 | 3 | 138,7 | 6 | 113,7 |
| 2 | 6 | 113,7 | 4 | 110,5 | 5 | 112,6 |
| 3 | 5 | 112,6 | 7 | 105,5 | 2 | 105,0 |
| 4 | 4 | 110,5 | 1 | 98,3 | 8 | 98,5 |
| 5 | 7 | 105,0 | Mitt-lere Be-wertung | 113,25 | Mitt-lere Be-wertung | 107,45 |
| 6 | 2 | 105,0 | | | | |
| 7 | 8 | 98,5 | | | | |
| 8 | 1 | 98,3 | | | | |

Am nächsten Tag (Tag 0) wurde jedem der Pferde eine Injektion zwischen 7,5 ml und 9,5 ml, je nach dem Körpergewicht, in den Halsmuskel gegeben. Jedes Pferd erhielt eine auf den nächsten Wert von 0,5 ml gerundete Injektion von 0,0176 ml/kg (0,008 ml/lb.), bezogen auf das Körpergewicht. Die Injektion für die Pferde der Behandlungsgruppe bestand aus einer sterilen wäßrigen Lösung mit 1,19 Gew.-% Natriumhyaluronat mit einem mittels FPLC bestimmten mittleren Molekulargewicht von $1,88 \times 10^5$, einem Nucleinsäure-Gehalt von weniger als 0,003 mg/ml mittels Ethidiumbromid-Fluoreszenz, einem Gesamt-Aminosäure-Gehalt mittels o-Phthalaldehyd-Fluoreszenz von weniger als 0,005 mg/ml und einer Viskosität von 147 cSt bei 37 °C, während die Injektion für die Pferde der Kontrollgruppe aus einer sterilen phosphatgepufferten Kochsalz-Lösung bestand. Das Gewicht jedes Pferdes und die ihm zu diesem Zeitpunkt und vier und acht Tage

danach verabreichte Dosis waren wie folgt:

| Pferd Nr. | Gewicht | | Dosis |
|---|---|---|---|
| | kg | (lb.) | ml |
| 1 | 540 | (1190) | 9,5 |
| 2 | 460 | (1015) | 8,0 |
| 3 | 447 | (985) | 8,0 |
| 4 | 551 | (1215) | 9,5 |
| 5 | 467 | (1030) | 8,0 |
| 6 | 549 | (1210) | 9,5 |
| 7 | 429 | (945) | 7,5 |
| 8 | 535 | (1180) | 9,5 |

Die Injektionsstelle wurde abgetastet, und die Körpertemperatur wurde gemessen, und zwar jeweils täglich drei Tage nach der ersten Injektion, zum Zeitpunkt der zweiten Injektion und zum Zeitpunkt der dritten Injektion sowie schließlich vier Tage nach der dritten Injektion. Die IM-Behandlung verursachte keine schädlichen Wirkungen. Sämtliche Temperaturen blieben normal, und es gab keine klinisch signifikante Reaktion an der Injektionsstelle (das Freund'sche Complete Adjuvans ist ein "bekanntes Pyrogen", so daß die Normalität der Temperatur aufgrund der Tatsache beurteilt wurde, daß die Behandlungs-Injektionen keinen weiteren Temperaturanstieg bewirkten und den Temperaturrückgang von dem durch die traumatisierende Injektion induzierten Peak-Wert nicht zu stören schienen.

Nach den Kriterien Gelenkumfang, Bewegungsbereich, Länge des Ausschreitens und beobachtete Lahmheit wurde jedes Pferd 7, 14, 21, 28, 35 und 42 Tage nach der ersten Injektion bewertet. Hierbei handelte es sich um die gleichen Parameter, die zur Einteilung der Pferde in die Behandlungsgruppe und die Kontrollgruppe herangezogen worden waren.

Durchführung der Bewertung

1. Gelenkumfang

Der Gelenkumfang wurde gemessen, während sich das Tier vor der Arbeit noch in der Box befand. Er wurde an einem Punkt unmittelbar über dem akzessorischen Karpalknochen gemessen. Er wurde mit einem Tuchband gemessen und in cm aufgezeichnet.

2. Bewegungsbereich

Der Bewegungsbereich wurde in der Box gemessen. Er ist gegeben durch die Differenz zwischen dem Winkel des erkrankten Beines in Ruhe und dem Biegungswinkel. Alle drei Werte wurden aufgezeichnet. Zur Bestimmung der Winkel wurde ein Goniometer verwendet.

a. Bein in Ruhe: An dem Pferd in stehender Position wurde mittels des Goniometers der Winkel des Carpus gemessen.

b. Biegungswinkel: Das erkrankte Gelenk wurde gebogen, während das Bein vom Boden abgehoben war. Mittels des Goniometers wurde der Winkel gemessen, bei dem das Pferd auf das Biegen durch Ausweichen, scheuen oder Zurückziehen reagierte.

3. Länge des Ausschreitens

Die schrittlänge wurde gemessen, bevor das Pferd auf eine Gehmaschine gestellt wurde. Eine lange Papierrolle (6 m; 20 feet) wurde zum Aufzeichnen des Abstandes zwischen den Markierungen der Hufe verwendet. Vor dem Gehen über das Papier wurde der Huf des erkrankten Beines mit Wasser oder Mineralöl bestrichen. Zwei Schritte wurden aufgezeichnet, und der Abstand zwischen Huf-Markierungen des erkrankten Beines wurde in cm gemessen. Das Pferd wurde dreimal über das Papier gehen gelassen. Ein Mittelwert aus den drei Messungen wurde als Endwert eingesetzt.

4. Beobachtete Lahmheit

Das Pferd wurde auf die mechanische Gehmaschine gestellt und 5 min mit einer Geschwindigkeit von 9,6 km\h (6 m.p.h.) gehen gelassen. Die Bewertung wird vorgenommen, während das Pferd sich auf der Gehmaschine befindet. Die Richtung der mechanischen Gehmaschine wurde so eingestellt, daß das erkrankte Bein sich auf der Innenseite befand.

0 = keine Lahmheit.

1 = schwierig zu beobachten; nicht durchgängig auftretend, unabhängig von den Umständen (d.h. Gewicht tragend, im Kreis gehend, schief, harte Oberfläche etc.).

2 = schwierig zu beobachten beim Gehen oder Traben in gerader Richtung; durchgängig auftretend unter bestimmten Umständen (d.h. Gewicht tragend, im Kreis gehend, schief, harte Oberfläche etc.).

3 = durchgängig auftretend beim Traben unter allen Bedingungen.

4 = offensichtliche Lahmheit, ausgeprägtes Nicken, Hinken oder verkürzter Schritt.

5 = minimale Gewichtsbelastung in Bewegung und/oder in Ruhe; Unfähigkeit, sich zu bewegen.

Wenn ein Pferd sich der Bewegung sehr widersetzte und ein Plazieren auf der mechanischen Gehmaschine nicht ratsam war, wurde eine Bewertung von 5 verzeichnet, und das Pferd wurde in die Box zurückgebracht.

Eine Analyse dieser Parameter zeigte deutlich das günstige Ergebnis der intramuskulären Hyaluronsäure-Injektionen auf die Linderung des durch das Freund'sche Complete Adjuvant induzierte Trauma. Die Pferde der Behandlungsgruppe zeigten gegenüber den Pferden der Kontroll-Gruppe ein deutlich überlegenes Verhalten im Test in allen Parametern mit Ausnahme des Gelenkumfangs, für den Ergebnisse nicht so schlüssig waren. Dies steht im Einklang mit anderen Untersuchungen über die Besserung von Traumata in Pferdegelenken, in denen gefunden wurde, daß der Gelenkumfang kein besonders empfindlicher Meßparameter ist. Die Ergebnisse sind in der nachstehenden Tabelle zusammengestellt, in denen "Tag" die Zahl der Tage vor oder nach der ersten Injektion bezeichnet:

## Tabelle

**Parameter: Gelenkumfang (in cm)**

| Tag | Behandlungsgruppe Pferd Nr. | | | | Kontrollgruppe Pferd Nr. | | | |
|---|---|---|---|---|---|---|---|---|
| | 8 | 2 | 5 | 6 | 3 | 4 | 1 | 7 |
| -6 | 31,0 | 29,4 | 29,8 | 31,5 | 29,8 | 30,0 | 31,9 | 30,5 |
| -1 | 34,5 | 34,5 | 33,4 | 36,2 | 33,5 | 35,5 | 36,2 | 36,0 |
| 7 | 36,4 | 33,5 | 37,2 | 36,6 | 35,7 | 35,5 | 37,0 | 36,6 |
| 14 | 36,7 | 34,5 | 36,3 | 35,5 | 36,8 | 36,4 | 36,8 | 36,8 |
| 21 | 36,1 | 35,6 | 37,7 | 35,8 | 36,8 | 36,3 | 37,5 | 37,7 |
| 28 | 35,6 | 33,8 | 35,5 | 34,0 | 37,2 | 35,3 | 37,5 | 36,4 |
| 35 | 35,2 | 34,2 | 36,2 | 35,3 | 36,3 | 36,5 | 39,4 | 39,2 |
| 42 | 35,8 | 34,0 | 35,5 | 34,5 | 39,3 | 34,0 | 38,8 | 40,0 |

**Parameter: Bewegungsbereich**

| Tag | Behandlungsgruppe Pferd Nr. | | | | Kontrollgruppe Pferd Nr. | | | |
|---|---|---|---|---|---|---|---|---|
| | 8 | 2 | 5 | 6 | 3 | 4 | 1 | 7 |
| -6 | 150° | 150° | 150° | 140° | 160° | 145° | 155° | 150° |
| -1 | 60° | 55° | 45° | 35° | 30° | 45° | 65° | 55° |
| 7 | 40° | 75° | 65° | 85° | 50° | 50° | 85° | 85° |
| 14 | 70° | 95° | 70° | 115° | 45° | 55° | 75° | 85° |
| 21 | 95° | 95° | 70° | 130° | 40° | 60° | 95° | 55° |
| 28 | 100° | 110° | 70° | 110° | 55° | 80° | 90° | 75° |
| 35 | 100° | 95° | 75° | 115° | 75° | 75° | 75° | 75° |
| 42 | 95° | 100° | 70° | 105° | 55° | 85° | 75° | 55° |

- wird fortgesetzt -

## Parameter: Lahmheit

| | Behandlungsgruppe Pferd Nr. | | | | Kontrollgruppe Pferd Nr. | | | |
|---|---|---|---|---|---|---|---|---|
| | 8 | 2 | 5 | 6 | 3 | 4 | 1 | 7 |
| Tag | | | | | | | | |
| -6 | 0 | 0 | 0 | 0 | 0 | 0 | 0 | 0 |
| -1 | 5 | 5 | 4 | 4 | 5 | 5 | 4 | 5 |
| 7 | 4 | 3 | 4 | 1 | 4 | 5 | 4 | 4 |
| 14 | 1 | 1 | 3 | 0 | 4 | 4 | 2 | 4 |
| 21 | 0 | 1 | 3 | 0 | 4 | 4 | 4 | 4 |
| 28 | 1 | 1 | 2 | 0 | 4 | 3 | 4 | 4 |
| 35 | 0 | 0 | 0 | 0 | 4 | 1 | 3 | 3 |
| 42 | 0 | 0 | 0 | 0 | 4 | 1 | 1 | 3 |

## Parameter: Schrittlänge in cm (in inches)

| | Behandlungsgruppe Pferd Nr. | | | | Kontrollgruppe Pferd Nr. | | | |
|---|---|---|---|---|---|---|---|---|
| | 8 | 2 | 5 | 6 | 3 | 4 | 1 | 7 |
| Tag | | | | | | | | |
| -6 | 393 | 410 | 414 | 410 | 400 | 407 | 401 | 369 |
| | (154,9) | (161,5) | (163,1) | (161,3) | (157,3) | (160,4) | (157,7) | (145,4) |
| -1 | 291 | 193 | 322 | 370 | 189 | 0* | 354 | 256 |
| | (114,7) | (76,1) | (126,7) | (145,7) | (74,5) | (0) | (139,5) | (100,9) |
| 7 | 347 | 378 | 345 | 379 | 271 | 277 | 369 | 362 |
| | (136,7) | (148,8) | (135,8) | (149,2) | (106,5) | (108,9) | (145,2) | (142,5) |
| 14 | 376 | 394 | 399 | 411 | 314 | 363 | 416 | 339 |
| | (148,2) | (155,0) | (156,9) | (161,8) | (123,6) | (142,9) | (163,8) | (133,3) |
| 21 | 368 | 369 | 390 | 427 | 326 | 365 | 408 | 368 |
| | (144,9) | (145,3) | (153,4) | (168,2) | (128,5) | (143,7) | (160,7) | (144,9) |
| 28 | 378 | 400 | 395 | 418 | 330 | 386 | 386 | 354 |
| | (149,0) | (157,4) | (155,4) | (164,7) | (130,0) | (152,1) | (152,0) | (139,3) |
| 35 | 380 | 398 | 406 | 420 | 282 | 374 | 419 | 404 |
| | (149,5) | (156,8) | (159,8) | (165,2) | (111,2) | (147,1) | (164,9) | (159,0) |
| 42 | 391 | 413 | 430 | 424 | 326 | 385 | 419 | 371 |
| | (154,0) | (162,6) | (169,3) | (167,1) | (128,2) | (151,7) | (165,0) | (145,9) |

* Schrittmessung unmöglich; Pferd setzte kranken Fuß nicht auf Papier

Analysen dieser Ergebnisse wurden für jeden der Parameter zusammen mit einigen Daten der intraartikulären Behandlung der Gelenke von Pferden, die in ähnlicher Weise durch Injektion von Freund's Complete Adjuvant traumatisiert worden waren, aufgezeichnet. Die induzierten Traumata waren jedoch etwas weniger schwer als diejenigen der vorliegenden Erfindung, so daß die Kontroll-Pferde aus dieser Untersuchung einen Bewegungsbereich hatten, der sich nicht zu sehr von den Behandlungs-Pferden der vorliegenden Untersuchung unterschied. Wichtiger ist jedoch, daß der Unterschied in der Wiedererlangung des Bewegungsbereichs und der Rückgang des Lahmens zwischen den Behandlungs- und den Kontroll-Pferden für die vorliegende Untersuchung eng parallel zu demjenigen verlief, der bei der früheren Untersuchung beobachtet wurde. Somit ähnelten die durch intramuskuläre Injektion erzielten Effekte in starkem Maße denjenigen, die vorher durch intraartikuläre Verabreichung erzielt worden waren.

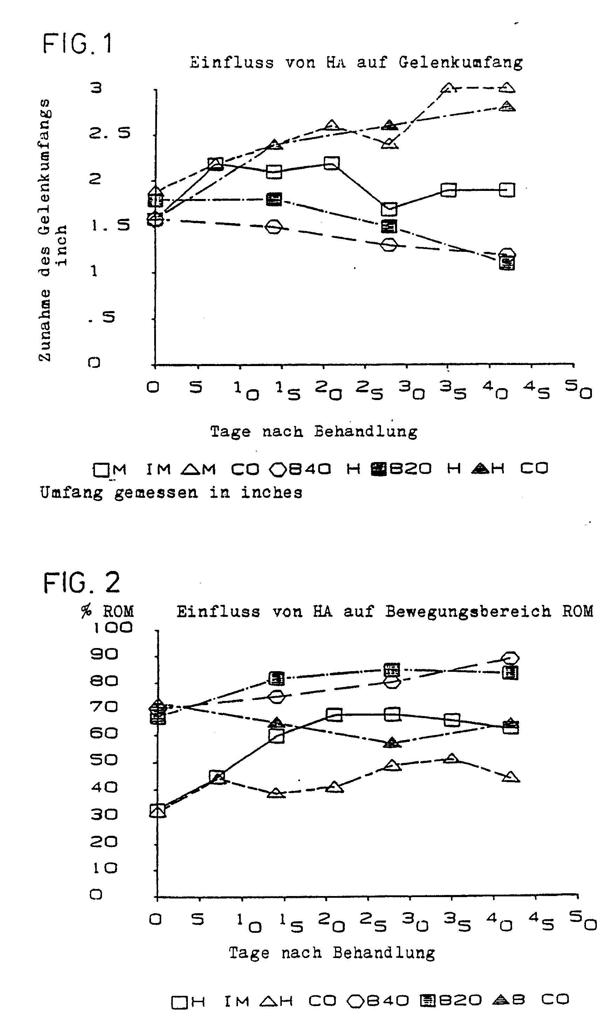

Die Analysen sind in den Fig. 1 bis 6 dargestellt. In diesen Abbildungen bezeichnen die leeren Quadrate die Mittelwerte für die vier Behändlungs-Pferde, und die leeren Dreiecke bezeichnen die Mittelwerte für die vier Kontroll-Pferde dieser Untersuchung. Die Sechsecke und die schraffierten Quadrate bezeichnen die Mittelwerte für Pferde mit einem ähnlich induzierten Gelenek-Trauma, die intra-artikulär mit einer Einzeldosis von 40 mg bzw. 20 mg einer ähnlichen wäßrigen Natriumhyaluronat-Lösung behandelt wurden, während die schraffierten Dreiecke die Mittelwerte für die in dieser früheren Untersuchung herangezogenen, unbehandelten Kontroll-Pferde bezeichnen.

Fig. 1 zeigt die Ände rungen des "Gelenkumfangs" vom Tage vor der ersten Injektion bis zum Ende der Untersuchung 43 Tage später {aus Gründen der Vereinfachung ist der "Tag-1" auf der 0-Achse aufgetragen und stellt die Zunahme des Gelenkumfangs (in inches) seit dem Tag vor der traumatisierenden Injektion, d.h. dem Tag -6, dar}. Obwohl die Gelenkschwellung niemals vollständig reversibel verläuft, wird sie durch die Hyaluronsäure-Behandlung auf einem niedrigen Niveau stabilisiert oder teilweise gelindert. Der Effekt ist etwas weniger dramatisch bei dem vorliegenden (intramuskulären) Verabreichungsweg, jedoch ist er eindeutig vorhanden und zeigt sich an Gelenken, für die durch die anderen Parameter indiziert war, daß sie schwerer traumatisiert waren als diejenigen, die intra-artikulär behandelt wurden.

Fig. 2 zeigt die Änderungen des "Bewegungsbereichs" (ROM) während des gleichen Zeitraums wie in Fig. 1. Der Wert 100 % basiert auf dem am Tag vor der Traumatisierung, d.h. dem Tag -6, gefundenen ROM. Es wird deutlich, daß die in der intra-artikulären Vergleichsstudie untersuchten Gelenke weniger stark traumatisiert waren, da die Behinderung des ROM vor der Behandlung weniger schwer war.

Es wird ebenfalls deutlich, daß die intramuskuläre Behandlung und die intra-artikuläre Behandlung parallele Verbesserungen gegenüber ihren jeweiligen Kontrollen bewirkt haben. Da jedoch Freund's Complete Adjuvant ein extremes Trauma verursacht, waren weder die erfindungsgemäße Behandlung noch die Vergleichsbehandlung in der Lage, an induzierten Zustand vollständig zu lindern.

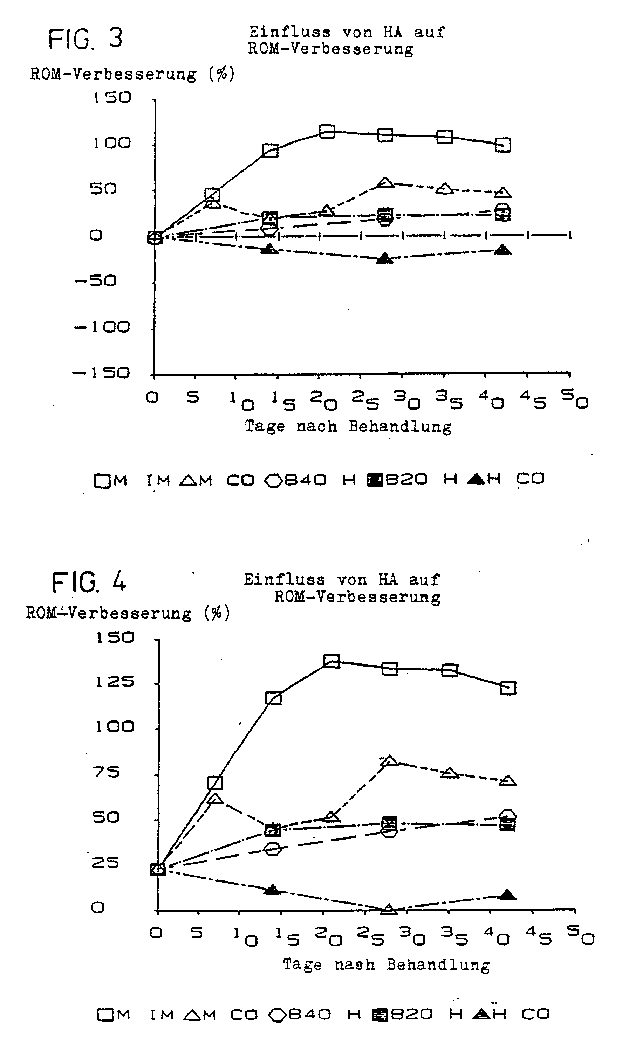

Fig. 3 und Fig. 4 zeigen weitere Analysen der ROM-Daten, in denen die prozentuale Verbesserung gegenüber dem Zustand an dem Tag vor der ersten Injektionsbehandlung ausgewertet werden. Die Zeit-Skala ist die gleiche wie in Fig. 1 und Fig. 2. In Fig. 4 ist die graphische Darstellung willkürliche durch Addition von 23,7 % zu allen Ordinatenwerten nach oben verschoben worden. Hier sieht es so aus, daß die intramuskuläre Verabreichung gemäß der vorliegenden Erfindung gegenüber ihrer Kontrolle eine größere Verbesserung ergibt, als dies bei der intra-artikulären Verabreichung der Fall ist.

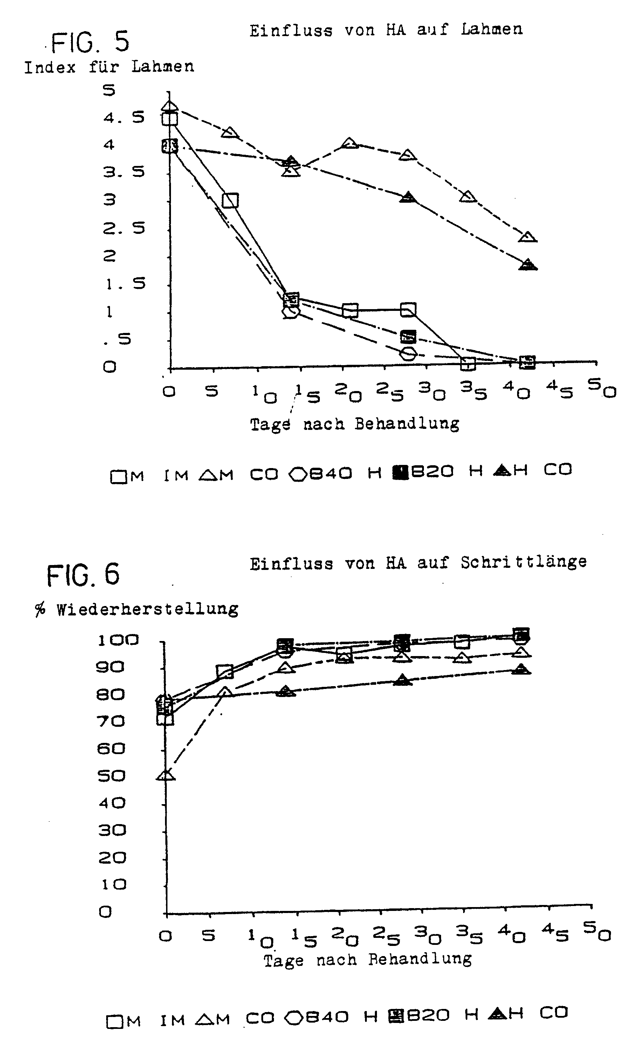

Fig. 5 zeigt die Änderung der "beobachteten Lahmheit" während des gleichen Zeitraums wie die vorhergehenden Figuren. Der intramuskuläre Verabreichungsweg und der intra-artikuläre Verabreichungsweg zeigten annähernd äquivalente Effekte. Die Kontrollen für die vorliegende Untersuchung zeigten einen etwas höheren Grad der Lahmheit, was andeutet, daß die Traumatisierung für die vorliegende Untersuchung etwas schwerer war als diejenige für die vorhergehende intra-artikuläre Untersuchung. Die Tatsache, daß der gleiche Wert der Lahmheit bei beiden Verabreichungswegen erreicht wurde, deutet an, daß der Weg gemäß der vorliegenden Erfindung etwas wirksamer sein könnte.

Fig. 6 zeigt die Änderung der "Schrittlänge" während des gleichen Zeitraums wie die vorhergehenden Figuren. Der Schritt wurde bei jeder Bewertung verglichen mit dem am Tag vor der traumatisierenden Injektion, d.h. dem Tag -6, beobachteten Mittelwert, wodurch ein Prozentsatz der Wiederherstellung erhalten wurde. Sowohl die gemäß der vorliegenden Erfindung behandelten Pferde als auch die mittels intra-artikulärer Injektion behandelten Pferde zeigen, verglichen mit ihrem jeweiligen Kontrollen, eine raschere Wiederherstellung und einen höheren Grad der endgültigen Wiederherstellung der Schrittlänge.

Beispiel 2

Rückenschmerzen bei einem Mann, Alter 48, Gewicht 70 kg (155 lbs.), wurden durch topische

EP 0 243 867 B1

Anwendung einer wäßrigen Lösung von Natriumhyaluronat, gemischt mit 10 bis 30 Vol.-% Dimethylsulfoxid (DMSO), gelindert. Im einzelnen wurden Präparate mit 10, 20 und 30 Vol.-% DMSO hergestellt, in denen der Rest jeweils aus einer Lösung von 1,7 Gew.-% Natriumhyaluronat mit einer Viskosität oberhalb von 500 cSt bei 37 °C und einem mittels FPLC bestimmten Molekulargewicht von $2 \times 10^6$ u ($2 \times 10^6$ Dalton) sowie eine m Nucleinsäure-Gehalt von 0,00137 mg/ml und einem Gesamt-Aminosäure-Gehalt, bestimmt durch o-Phthalaldehyd-Fluoreszenz, von 0,0047 mg/ml bestand. Applikationen von etwa 2 ml des Präparats erfolgten auf dem Rücken in der Nachbarschaft der schmerzenden Stelle zweimal täglich während eines Zeitraums von 2 Wochen. In jedem Fall wurde der Schmerz in etwa 15 min gelindert, und diese Linderung hielt etwa 8 bis 10 h an. Dann wurde die Behandlung zeitweise unterbrochen, und die Schmerzen traten nach etwa 4 bis 5 Tagen wieder auf. Zu dieser Zeit wurde die Behandlung durch tägliche Applikation von etwa 2 ml des Präparats auf die Handgelenke wieder aufgenommen, und eine wirksame Schmerzlinderung wurde erreicht. Diese Behandlung wurde nach drei Tagen unterbrochen, und die Schmerzen traten in den nächsten zweieinhalb Wochen nicht wieder auf. Demgemäß wurden die Schmerzen wirksam behandelt mit einer topischen Dosis von etwa 0,33 mg/kg Körpergewicht (0,15 mg/lb.) (2 ml x 1,7 Gew.-% x 70 Vol.-% x 1000 mg/ml), die entweder in der Nähe der schmerzenden Stelle oder an einem weiter entfernten Ort aufgetragen wurde.

Die auf diese Weise gelinderten Schmerzen waren vor Beginn der Behandlung etwa zwei Jahre lang nahezu konstant gewesen. Nach der Schmerzbehandlung wurde der Schmerz in viel geringerer Stärke nur nach dem Sitzen oder Schlafen während eines längeren Zeitraums in konstanter Position wahrgenommen.

Die topische Anwendung des Natriumhyaluronats ohne einen transdermalen Träger war unwirksam. Die Natriumhyaluronat-Lösung verdampfte einfach zur Trockne und hinterließ einen Film auf der Haut des Patienten.

Die Verabreichung äquivalenter Mengen DMSO allein war ebenfalls unwirksam in bezug auf eine Linderung der Rückenschmerzen. Tatsächlich verursachte sie eine gewisse Reizung der Fläche der Haut, auf die das DMSO aufgebracht worden war. Dies stand in deutlichem Gegensatz zu der Anwendung in Kombination mit der Natriumhyaluronat-Lösung, bei der keine Hautreizung beobachtet wurde.


Beispiel 3

Verschiedene Gelenk- und Muskel-Schmerzen wurden bei einem Mann, Alter 54, Gewicht 113 kg (250 lbs.), durch topische Anwendung der gleichen wäßrigen Natriumhyaluronat-Lösung, wie sie in Beispiel 2 verwendet wurde, kombiniert mit 20 Vol.-% DMSO, bezogen auf das Gesamt-Volumen, gelindert. Ein Knie, das infolge eines mehrere Jahre alten Knorpel-Schadens Schmerzen bereitete, wurde mit 4 ml dieses Präparats behandelt, und innerhalb von 30 min wurde Erleichterung verspürt. Die Behandlung wurde in vier Tagesintervallen wiederholt, wodurch die Schmerzen erfolgreich etwa zwei Wochen lang bekämpft wurden. Bursitis-Schmerzen in einer Schulter wurden erfolgreich innerhalb weniger Minuten nach topischer Anwendung von etwa 5 ml desselben Präparats gelindert. Die Schmerzen kehrten nach etwa vier Tagen zurück und wurden durch die gleiche Behandlung gelindert. Der Cyclus wurde viermal wiederholt. Schmerzen von wunden Muskeln in der Schlüsselbein-Gegend wurden durch Anwendung von 3 ml dieses Präparats gelindert. Die Applikation erfolgte nur auf einer Seite, und die Schmerzen bestanden auf der anderen Seite fort.


Beispiel 4

Schwere Rücken-Schmerzen bei einem Mann, Alter 57, Gewicht 88,5 kg (195 lbs.), der unter mehrfachem Bandscheibenvorfall in der Lendengegend mit dadurch bedingten Muskelkrämpfen litt, wurden durch die topische Anwendung von 2 ml des in Beispiel 2 beschriebenen Präparats mit 20 Vol.-% DMSO gelindert. Das Präparat wurde auf die Haut über der Lendenwirbelsäule aufgebracht und linderte die Schmerzen innerhalb einer Stunde für die Dauer von etwa acht Stunden. Die Behandlung wurde in 12-Stunden-Intervallen während eines Zeitraums von mehreren Tagen durchgeführt, wobei gelegentlich eine Behandlung ausgelassen wurde. Bei manchen Gelegenheiten war die Behandlung unwirksam, jedoch war in solchen Fällen die nachfolgende Behandlung wirksam.

Der der Behandlung unterzogene Zustand war vorher mehr als ei n Jahr durch Enzym-Injektionstherapie behandelt worden. Zum Zeitpunkt des Beginns der vorliegenden Behandlung wurde ein elektiver chirurgischer Eingriff erwogen.

14

Beispiel 5

Zwei Quarter-Horse-Reitpferde, die normalerweise aktiv an Tonnen-Rennen (barrel races) teilnahmen, entwickelten Tendonitis der Flexor-Sehnen beider Hinterbeine und wurden erfolgreich behandelt durch topische Applikation der wäßrigen Natriumhyaluronat-Lösung des Beispiels 2 im Gemisch mit einem transdermalen Träger. Die Mischung aus 80 Vol.-% Natriumhyaluronat/20 Vol.-% Dimethylsulfoxid war wirksamer als ein im Handel erhältliches topisches Mittel, Absorbine ®. Ein Bein jedes Pferdes wurde mit der Hyaluronat-Mischung behandelt, und das andere Bein wurde mit Absorbine behandelt. Das Hyaluronat wurde zweimal täglich drei Tage lang appliziert, wobei Auftragungen von 3 ml am ersten Tag und Auftragungen von 1 ml an den beiden nachfolgenden Tagen angewandt wurden (in jedem Falle wurde jeweils die Hälfte jeder Auftragung auf jeweils einer der beiden Seiten des Beins aufgebracht). Am Ende der drei Tage waren die Symptome, Schwellung über der Sehne und Scheide und wunde Stellen über den Sesambein-Knochen, beseitigt. Die primäre Läsion war verschwunden. Andererseits erforderte die Behandlung mit Absorbine in der empfohlenen Weise sechs Behandlungstage bis zur vollen Besserung.

Vergleichsbeispiel 1

Zwei Versuche wurden durchgeführt, die eine Begrenzung bei der Fernverabreichung der Hyaluronsäure aufzeigten. Zwei der Kontroll-Pferde aus der Untersuchung, über die in Beispiel 1 berichtet wurde, wurden topisch behandelt, und zwei andere wurden intravenös behandelt, nachdem ihre Traumata chronisch geworden waren und keine signifikante Besserung ihres jeweiligen Zustands mehr beobachtet wurde. Obwohl in der EP-A-0 144 019 über eine gewisse Linderung des durch Freund's Complete Adjuvant induzierten chronischen Zustandes durch intra-artikuläre Injektion von Natriumhyaluronat berichtet wurde, ist dies Modell im allgemeinen auf die Untersuchung der Behandlung akuter Zustände beschränkt. Die längere Zeit nach der Traumatisierung entwickelten Läsionen sind so schwer, daß normalerweise nicht erwartet wird, daß sie auf die Behandlung ansprechen. Aufgrunddessen werden diese Ergebnisse nicht als ernsthafte Einschränkung der Fernverabreichung gewertet und können tatsächlich eher so eingestuft werden, daß sie die Schwere des induzierten Traumas widerspiegeln.

Die topische Behandlung erfolgte einmal täglich sechs Tage lang, beginnend am "Tag 28" der Untersuchung des Beispiels 1, d.h. 34 Tage nach der Induzierung des Traumas, durch Aufbringen von 10 ml einer Mischung aus 80 Vol.-% Natriumhyaluronat/20 Vol.-% Dimethylsulfoxid auf das erkrankte Gelenk. Die wäßrige Hyaluronat-Lösung war die gleiche, wie sie auch in Beispiel 2 verwendet wurde. Keine signifikante Verbesserung bei irgendeinem der vier Kriterien von Beispiel 1 wurde beobachtet, und zwar sowohl im Vergleich zu dem jeweiligen Zustand unmittelbar vor der Behandlung als auch im Vergleich zu den beiden anderen Kontroll-Pferden.

Die intravenöse Behandlung erfolgte jeden zweiten Tag acht Tage lang beginnend mit dem "Tag 35" der Untersuchung des Beispiels 1, d.h. 41 Tage nach der Induzierung des Traumas, durch Injektion von 4 ml der in Beispiel 1 eingesetzten wäßrigen Natriumhyaluronat-Lösung in die juguläre Vene. Wiederum wurde keine signifikante Besserung bei irgendeinem der vier Kriterien beobachtet.

**Ansprüche**

1. Verwendung von Hyaluronsäure oder eines pharmakologisch unbedenklichen Salzes derselben zur Herstellung von intravenös, intramuskulär oder topisch verabreichbaren Arzneimitteln zur Behandlung von Arthritis in Gelenken von Pferden und Menschen.

2. Verwendung nach Anspruch 1, dadurch gekennzeichnet, daß für die Herstellung einer Injektionslösung die Hyaluronsäure in Form einer wäßrigen Lösung von 0,5 bis 3,0 Gew.-% mit einer Viskosität von weniger als etwa 200 cST bei 37° C eingesetzt wird.

3. Verwendung nach Anspruch 1, dadurch gekennzeichnet, daß die Hyaluronsäure eine mittels FPLC bestimmte Molekulargewichtsverteilung aufweist, die nahezu vollständig zwischen 1,5 und $4 \times 10^6$ u (1,5 und $4 \times 10^6$ Dalton) liegt.

4. Verwendung nach Anspruch 1, dadurch gekennzeichnet, daß die Hyaluronsäure nicht pyrogen ist und einen mittels UV-Extinktion bestimmten kombinierten Aminosäure- und Protein-Gehalt von weniger als etwa 1,25 mg/ml und einen Nucleinsäure-Gehalt von weniger als etwa 0,06 mg/ml aufweist.

## Claims

1. Use of hyaluronic acid or a pharmacologically acceptable salt thereof for the production of medicaments which can be administered intravenously, intramuscularly or topically for the treatment of arthritis in joints of horses and humans.

2. Use according to Claim 1, characterized in that for the production of an injection solution the hyaluronic acid is employed in the form of an aqueous solution of 0.5 to 3.0% by weight having a viscosity of less than about 200 cST at 37° C.

3. Use according to Claim 1, characterized in that the hyaluronic acid has a molecular weight distribution determined by means of FPLC, which is nearly completely between 1.5 and 4 x $10^6$ u (1.5 and 4 x $10^6$ Dalton).

4. Use according to Claim 1, characterized in that the hyaluronic acid is non-pyrogenic and has a combined amino acid and protein content determined by means of UV extinction of less than about 1.25 mg/ml and a nucleic acid content of less than about 0.06 mg/ml.

## Revendications

1. Utilisation d'acide hyaluronique ou d'un sel pharmacologiquement acceptable de cet acide pour la préparation de médicaments pouvant être administrés par voie intraveineuse, intramusculaire ou localement pour le traitement de l'arthrite affectant des articulations de chevaux et d'êtres humains.

2. Utilisation suivant la revendication 1, caractérisé en ce que, pour la préparation d'une solution injectable, on utilise l'acide hyaluronique sous forme d'une solution aqueuse à 0,5-3,0 % en poids ayant une viscosité de moins d'environ 200 cST à 37° C.

3. Utilisation suivant la revendication 1, caractérisé en ce que l'acide hyaluronique présente une répartition de poids moléculaire, déterminée par FPLC, qui se situe à peu près totalement entre 1,5 et 4x$10^6$ u (1,5 et 4x$10^6$ daltons).

4. Utilisation suivant la revendication 1, caractérisée en ce que l'acide hyaluronique est non pyrogène et présente une teneur combinée en amino-acides et en protéine, déterminée par extinction UV, de moins d'environ 1,25 mg/ml et une teneur en acide nucléique de moins d'environ 0,06 mg/ml.

## FIG. 1

Einfluss von HA auf Gelenkumfang

Tage nach Behandlung

□M  IM  △M  CO  ○840  H  ■820  H  ▲H  CO

Umfang gemessen in inches

## FIG. 2

% ROM   Einfluss von HA auf Bewegungsbereich ROM

Tage nach Behandlung

□H  IM  △H  CO  ○840  ■820  ▲B  CO

FIG. 3

Einfluss von HA auf
ROM-Verbesserung

ROM-Verbesserung (%)

Tage nach Behandlung

☐M IM △M CO ◯840 H ▦820 H ▲H CO

FIG. 4

Einfluss von HA auf
ROM-Verbesserung

ROM-Verbesserung (%)

Tage nach Behandlung

☐M IM △M CO ◯840 H ▦820 H ▲H CO

18

## FIG. 5

Einfluss von HA auf Lahmen

Index für Lahmen

Tage nach Behandlung

□M IM △M CO ○840 H ▨820 H ▲H CO

## FIG. 6

Einfluss von HA auf Schrittlänge

% Wiederherstellung

Tage nach Behandlung

□M IM △M CO ○840 H ▨820 H ▲H CO